# EUROPEAN PATENT APPLICATION

(11) **EP 2 369 016 A1**
(43) Date of publication of application: **28.09.2011**
(21) Application number: 11168800.8
(22) Date of filing: 25.05.2007
(51) Int. Cl.: C12Q 1/68

(54) **Methods for diagnosing and treating graft rejection and inflammatory conditions**

(30) Priority: 25.05.2006 US 808122 P
(62) Divisional of application: 07729529.3
(71) Applicant: Institut National de la Santé et de la Recherche Médicale (INSERM), 75654 Paris Cedex 13 (FR)
(72) Inventor: Brouard, Sophie, 44240 Sucé-sur-Erdre (FR); Soulillou, Jean-Paul, 44000 Nantes (FR); Braud, Christophe, 44000 Nantes (FR); Ashton-Chess, Joanna, 44000 Nantes (FR)
(74) Representative: Chajmowicz, Marion

(57) **Abstract**

The present invention relates to a method of diagnosis and/or prognosis of an inflammatory condition, which method comprises determining the expression level of TRIB1 in a biological sample of a patient, in particular in peripheral blood or in a biopsy of a diseased tissue. Methods for treating inflammatory conditions by modulating TRIB1 expression or activity are also described.

## Description

The invention relates the identification of TRIB1 as (i) a marker for the diagnosis, in particular in blood, and prognosis of inflammatory conditions, such as chronic graft rejection, and (ii) as a therapeutic target for these conditions.

### Technical background

More than 250 000 Europeans live with an organ transplant and 80 000 remain on transplant waiting lists. Increasing the life of a transplanted organ remains a major challenge in transplantation. Indeed, despite an increase in graft survival and a decrease in the risks of rejection, the leading cause of graft loss, chronic rejection (CR), is only poorly influenced by currently used immunosuppressors.

There remains a need for a reliable diagnostic or prognostic test applicable to a blood sample or a graft biopsy, to monitor and adapt the therapeutic treatment available to each patient. The identification of a new marker is also a major objective in the search for novel immunosuppressors.

In this context, the literature reports a few studies of transcriptome profiling in renal transplant biopsies (Flechner et al, 2004a; Scherer et al, 2003), and one study in the peripheral blood lymphocytes of renal transplant patients (Flechner et al, 2004b).

International patent application W02005/070086 also describes expression profiles of genes that are determinative of graft tolerant phenotypes (see also Brouard et al, 2005; Louis et al 2006; Baeten et al 2006).

### Summary of the invention

The inventors have now identified that TRIB1 ("Tribbles homolog 1 ") is a marker of chronic rejection in clinical renal transplantation, and more generally of any inflammatory condition, such as joint inflammation, multiple sclerosis, and inflammatory bowel disease. More particularly the inventors showed that TRIB1 is a peripheral blood marker of such pathologies.

This new marker can serve to distinguish CR from other types of chronic dysfunction, thereby enabling therapeutic adaptation and more individualized immunosuppression. It further enables earlier detection of CR and thus improves graft outcome by allowing for early therapeutic intervention before the onset of irreversible graft damage.

On this basis, the invention provides an *in vitro* method of diagnosis and/or prognosis of an inflammatory condition, which method comprises determining the expression level of TRIB1 in a biological sample of a patient, wherein a higher expression of TRIB1 in the patient, compared to a reference value, is indicative of an inflammatory condition.

TRIB1 marker is all the more useful that its expression level can be easily assessed from any blood or plasma sample, as well as in tissue biopsies, using standard kits.

The expression level may be determined by any method known in the art, e.g. by measuring the level of TRIB1 mRNA in the biological sample, or by measuring the level of TRIB1 protein, e.g. by an immunoassay of the TRIB1 protein.

The invention further provides a method for screening compounds useful for treating an inflammatory condition, which method comprises determining the ability of a test compound to modulate the expression or activity of TRIB1. More particularly, the test compound that inhibits the expression or activity of TRIB1 is a candidate for treating an inflammatory condition.

Another subject of the invention is a method of treating an inflammatory condition, which method comprises modulating TRIB1 expression or activity.

In a preferred embodiment, this method comprises administering to a patient in need thereof a modulator of TRIB1 expression or activity selected from the group consisting of an antagonist of TRIB1, an antisense or a RNAi of TRIB1, and an antibody or a fragment or a derivative thereof specific to a TRIB1 polypeptide.

### Brief description of the drawings

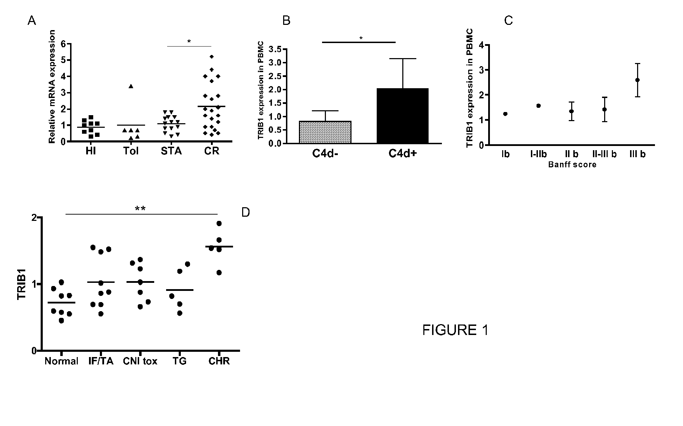

Figures 1A, 1B and 1C show the expression of TRIB1 as measured by quantitative RT-PCR. Figure 1A shows the expression level of TRIB1 in the peripheral blood mononuclear cells (PBMCs) of healthy individuals (HI) and different cohorts of renal transplant patients (TOL: tolerant; STA: stable graft function; CR: chronic rejection). Figure 1B shows the expression level of TRIB1 in PBMC of chronic rejection patients according to the presence of C4d staining within the biopsy from the same patients. Figure 1C shows the expression level of TRIB1 in PBMC of chronic rejection patients according to the Banff score of their biopsies. Figure 1D shows TRIB1 expression in graft biopsies from patients with chronic humoral rejection vs. other causes of late graft failure.

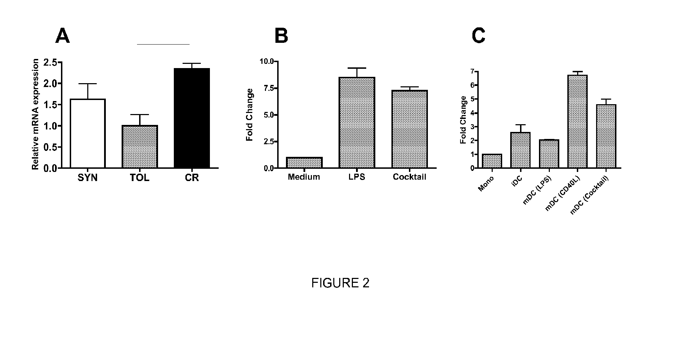

Figures 2A, 2B and 2C show the expression of TRIB1 in syngeneic (SYN), tolerating (TOL) or chronically rejecting (CR) heart transplants 100 days following allotransplantation in the rat (Figure 1A), in resting monocytes or following activation with LPS or a pro-inflammatory cocktail (Figure 1 B) or in immature (iDC) or mature (mDC) dendritic cells (Figure 1 C), as measured by quantitative RT-PCR.

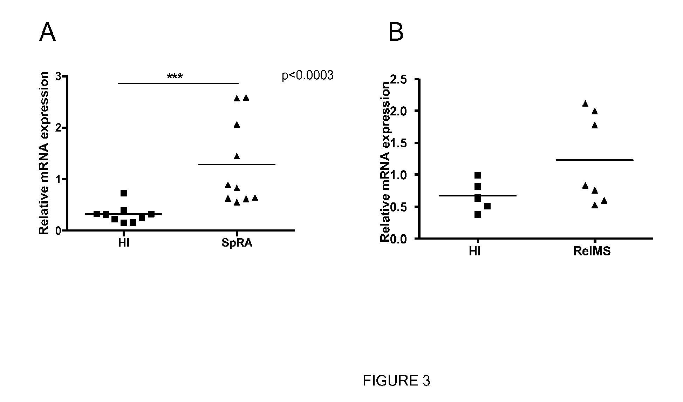

Figures 3A and 3B show the expression of TRIB1 in the PBMC of healthy individuals (HI) compared to patients suffering from Spondyloarthropathy (Figure 3A) or to patients suffering from relapsing Multiple Sclerosis (Figure 3B).

### Detailed description of the invention

TRIB1 can act as prognostic marker, thereby enabling the early detection of chronic rejection before the apparition of lesions within the organ transplant or chronic inflammatory conditions.

The invention provides methods for diagnosis or prognosis, as well as treatments, of an inflammatory condition.

"Inflammatory condition" refers to any inflammatory disease or disorder known in the art whether of a chronic or acute nature, including, but not limited to, rheumatoid arthritis, inflammatory skin diseases, such as, psoriasis and eczema, restenosis, multiple sclerosis, surgical adhesion, tuberculosis, inflammatory lung diseases such as asthma, pneumoconiosis, chronic obstructive pulmonary disease, nasal polyps and pulmonary fibrosis, inflammatory bowel disease, such as, Crohn's disease and ulcerative colitis, graft rejections, inflammatory diseases that affect or cause obstruction of a body passageway, such as, vasculitis, Wegener's granulomatosis and Kawasaki disease, systemic lupus erythematosus, inflammation of the eye, nose or throat, such as, neovascular diseases of the eye including neovascular glaucoma, proliferative diabetic retinopathy, retrolental fibroblasia, mascular degeneration, reduction of intraocular pressure, corneal neovascularization, such as, corneal infections, immunological processes, such as, graft rejection and Steven-Johnson's syndrome, alkali burns, trauma and inflammation (of any cause).

The inflammatory condition is more particularly a graft rejection, more particularly an allograft rejection. It can be a graft of any organ, in particular solid organs such as kidney, lung, liver, heart, etc. In a specific embodiment, the graft rejection is chronic rejection in clinical renal transplantation.

Most preferably, the condition is a chronic humoral rejection. Chronic humoral rejection (CHR) is characterized by transplant glomerulopathy accompanied by deposition of the complement split product C4d in graft biopsies and the presence of circulating anti-donor HLA antibody (Mauiyyedi S et al, 2001).

Other preferred inflammatory conditions include joint inflammation, multiple sclerosis, spondyloarthropathy or inflammatory bowel disease.

### Definitions

Tribbles homolog 1 (TRIB1) is a known gene which is a member of the Tribbles family that controls MAPK cascades (Kiss-Toth et al, 2004). SEQ ID NO:1 shows the cDNA sequence, and SEQ ID NO:2 shows the protein sequence, in humans. TRIB1 is referred to as a "marker" or "biomarker" in that it enables to diagnose or predict the development of an inflammatory condition.

The expression level of said marker is determined in a biological sample of a patient and compared to the expression level of said marker in a control subject.

The term "biological sample" means any biological sample derived from a patient, preferably a sample which contains nucleic acids. Examples of such samples include fluids, tissues, cell samples, organs, biopsies, etc. Most preferred samples are blood, plasma, saliva, urine, seminal fluid, tissue such as graft or diseased tissue biopsies etc. Peripheral blood or tissue biopsies are preferred, and mononuclear cells (PBMCs) are the preferred cells. Total RNAs can be easily extracted therefrom. The biological sample may be treated prior to its use, e.g. in order to render nucleic acids available. Techniques of cell or protein lysis, concentration or dilution of nucleic acids, are known by the skilled person.

The term "patient" refers to any subject to be tested. It is preferably a human, but includes any mammal, e.g. horses, cats, dogs, cattle, sheep, etc. The patient may be asymptomatic or may show early or advanced symptoms of an inflammatory condition.

The "diagnosis" means the identification of the condition or the assessment of the severity of the condition.

The term "prognosis" means the assessment of the outcome of the condition, i.e. to determine the evolution of the condition, and the risk of worsening.

The term "reference" generally refers to a control subject who is either healthy or is a subject who is not affected with an inflammatory condition but who may be optionally affected with a different condition. It may also be a statistic control value. In order to determine the evolution of the inflammatory condition, it may further be very useful to test a patient for the expression the marker, and to monitor the effect of a drug or the evolution of the condition, by testing him/her a second time, e. g. a few days later. In that case the results of the second test are compared with the results of the first test, and in general also with the results obtained with a "healthy" subject, i.e. a subject who is not affected with an inflammation or a subject who has not undergone grafting. The "reference" then refers either to the same test patient or to a "healthy subject".

"A higher expression of TRIB1", in the patient, compared to a reference value, means that the expression level of TRIB1 in the patient is significantly increased versus the reference value. The increase can be e.g. at least 5%, or at least 10%, or at least 20%, more preferably at least 30%.

### Determination of expression level of the markers

Determination of the expression level of a gene can be performed by a variety of techniques.

More preferably, the determination comprises contacting the sample with selective reagents such as probes, primers or ligands, and thereby detecting the presence, or measuring the amount, of polypeptide or nucleic acids of interest originally in the sample. Contacting may be performed in any suitable device, such as a plate, microtiter dish, test tube, well, glass, column, and so forth In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the sample.

In a preferred embodiment, the expression level may be determined by determining the quantity of mRNA. Generally the expression level is a relative expression level.

Methods for determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCI, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a particular embodiment of the invention, the method for diagnosis or prognosis of an inflammatory condition comprises the steps of providing total RNAs extracted from PBMCs obtained from a blood sample or tissue biopsy of the patient, and subjecting the RNAs to amplification and hybridization to specific probes.

Preferably the expression level is determined by real-time quantitative or semi-quantitative RT-PCR.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling.

The expression level of the TRIB1 marker may also be determined by assaying the marker protein. Various methods for detecting and/or quantifying the expression of the marker proteins are described in greater detail below.

Such methods comprise contacting a biological sample with a binding partner capable of selectively interacting with a marker protein present in the sample. The binding partner is generally an antibody, that may be polyclonal or monoclonal, preferably monoclonal.

Procedures for raising polyclonal antibodies are well known. Typically, such antibodies can be raised by administering the marker protein subcutaneously to New Zealand white rabbits which have first been bled to obtain pre-immune serum. Each injected material contains adjuvants with or without pulverized acrylamide gel containing the protein or polypeptide after SDS- polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed in E. Harlow, et. al., editors, Antibodies: A Laboratory Manual (1988), that further discloses procedures for preparing monoclonal antibodies.

Laboratory methods for preparing monoclonal antibodies are well known. Monoclonal antibodies (Mabs) may be prepared by immunizing the purified marker protein into a mammal, *e. g*. a mouse, rat, rabbit, goat, human. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody. Also contemplated is the use of Mabs produced by an expressing nucleic acid cloned from a hybridoma.

The presence of the marker protein can be detected using standard electrophoretic and immunodiagnostic techniques, including immunoassays such as competition, direct reaction, or sandwich type assays. Such assays include, but are not limited to, Western blots; agglutination tests; enzyme- labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound marker protein in a liquid phase from a solid phase support to which antigen-antibody complexes are bound. Solid supports which can be used in the practice of the invention include substrates such as nitrocellulose (e. g. , in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

Typically, a solid support is first reacted with a solid phase component (e.g., one or more anti-TRIB1 antibody) under suitable binding conditions such that the component is sufficiently immobilized to the support according to methods well known to those skilled in the art. After reacting the solid support with the solid phase component, any non-immobilized solid-phase components are removed from the support by washing, and the support-bound component is then contacted with a biological sample suspected of containing ligand moieties (*e. g*., TRIB1 molecules toward the immobilized antibodies) under suitable binding conditions. After washing to remove any non-bound ligand, a secondary binder moiety is added under suitable binding conditions, wherein the secondary binder is capable of associating selectively with the bound ligand. The presence of the secondary binder can then be detected using techniques well known in the art.

### Screening methods

The present invention also provides novel target and methods for the screening of drug candidates or leads. The methods include binding assays and/or functional assays, and may be performed in vitro, in cell systems, in animals, etc.

The invention thus provides a method for screening compounds useful for treating an inflammatory condition, which method comprises determining the ability of a test compound to modulate the expression or activity of TRIB1. Such method is typically performed *in vitro.*

In a particular embodiment of the methods of screening, the modulation is an inhibition.

Such tests of activity of the protein may include testing the ability of TRIB1 to phosphorylate and/or activate MAPK (see Kiss-Toth et al, 2004).

The screening methods of the invention may comprise preliminary steps of contacting in vitro a test compound with a TRIB1 gene or polypeptide and determining the ability of said test compound to bind said TRIB1 gene or polypeptide. Binding to said gene or polypeptide provides an indication as to the ability of the compound to modulate the activity of said target, and thus to affect a pathway leading to an inflammatory condition in a subject.

In a further particular embodiment, the method comprises contacting a recombinant host cell expressing a TRIB1 polypeptide according to the present invention with a test compound, and determining the ability of said test compound to bind said TRIB1 and to modulate the activity of TRIB1 polypeptide. The determination of binding may be performed by various techniques, such as by labeling of the test compound, by competition with a labeled reference ligand, etc.

The screening method of the invention may also comprise contacting in vitro a test compound with a TRIB1 polypeptide and determining the ability of said test compound to modulate the activity of said TRIB1 polypeptide.

A further object of this invention resides in a method of screening, said method comprising contacting in vitro a test compound with a TRIB1 gene and determining the ability of said test compound to modulate the expression of said TRIB1 gene.

In an other embodiment, this invention relates to a method of screening, said method comprising contacting a test compound with a recombinant host cell comprising a reporter construct, said reporter construct comprising a reporter gene under the control of a TRIB1 gene promoter, and selecting the test compounds that modulate expression of the reporter gene.

The above screening assays may be performed in any suitable device, such as plates, tubes, dishes, flasks, etc. Typically, the assay is performed in multi-wells plates. Several test compounds can be assayed in parallel. Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance.

### Therapeutic applications

It is further described a method of treating an inflammatory condition in a patient in need thereof, which method comprises modulating TRIB1 expression or activity in said patient.

The invention also relates to the use of a modulator of TRIB1 expression or activity for the manufacture of a medicament intended for the treatment of an inflammatory condition in a patient in need thereof.

In the context of the invention, the term "treating" or "treatment", as used herein, means reversing, alleviating, inhibiting the progress of, or preventing the condition to which such term applies, or one or more symptoms of condition.

According to the invention, the term "patient" or "patient in need thereof, is intended for a human or non-human mammal affected or likely to be affected with the inflammatory condition.

The term "modulator of TRIB1 expression or activity" means any compound or substance that is capable of regulating, more particularly inhibiting, i.e. diminishing, the expression of TRIB1 or the activity of the protein. Such modulators include the compounds identified by means of the screening methods described above. It can be an antagonist of TRIB1, an antisense or a RNAi of TRIB1, an antibody or a fragment or a derivative thereof specific to a TRIB1 polypeptide.

Such modulators may be administered orally, parenterally (including subcutaneous injections, intravenous, intramuscular, or infusion techniques), by inhalation spray, or rectally, in dosage unit formulations containing conventional non-toxic pharmaceutically-acceptable carriers, adjuvants and vehicles.

The below example illustrates the invention without limiting its scope.

### EXAMPLE 1: Transplantation

### Experimental protocol

All patients are informed of the study protocol and give informed consent. All animals are treated in accordance with standard European and Institutional Guidelines.

### Gene candidate selection

The inventors performed a meta-analysis by comparing a candidate gene list with those published in the literature by other groups having performed microarray studies in the context of renal transplantation (Flechner et al 2004a; Scherer et al 2003; Akalin et al 2001;Flechner et al 2004b;Donauer et al 2003). The published studies concern only rejection or stable function (not tolerance) and primarily involve graft biopsies and not blood. The aim of the present approach was to identify gene candidates present in the blood in the present study and also present in the biopsies in the studies in the literature. To make such a meta-analysis possible, more than 3000 genes have been first converted into a single identifier, the so-called « GENE ID », using the gene accession conversion tool available at the NIH website: http://david.abcc.ncifcrf.gov/.

### Establishing and screening of a "bank" of cDNA samples from several cohorts of renal transplant patients and healthy individuals

In order to further ascertain the relevance of the chosen gene candidates to chronic rejection, a bank of cDNA samples from blood was established to compare the expression of the genes in chronic rejection, not only to tolerance, but also to other outcomes (stable function, renal failure, chronic rejection without immunosuppression) and also to age-matched healthy individuals. For this purpose approximately 50 patients with the relevant kidney function and healthy individuals have been recruited, blood samples collected on EDTA tubes and RNA and cDNA prepared from PBMC (Ficoll PBMC preparation and TRIZOL RNA extraction). In parallel, cDNA was prepared from RNA extracted from graft biopsies taken from renal transplant patients with normal graft histology or different diagnoses of late graft failure such as chronic allograft nephropathy, calcineurin inhibitor toxicity or chronic humoral rejection. Expression of the chosen molecules have been measured by quantitative RT-PCR using TaqMan primer and probe sets. Cell sorting was performed by multicolor flow cytometry on the PBMC of 16 transplant patients and healthy individuals to obtain RNA and cDNA from monocytes, CD4 T cells, CD8 T cells, NK cells and B cells (using antibodies labeled with different fluorochromes enabling separation from a single blood sample using FACSAria).

### Results

The meta-analysis led to the identification of 14 genes common between at least two of the published studies. An additional gene, Tribbles homolog 1 (TRIB1), was significantly upregulated in CR biopsies in a published microarray study (Flechner et al, 2004a) as well as in the blood of CR patients in the present study. Expression of TRIB1 was measured by qPCR in the PBMC and peripheral blood cell subtypes of several cohorts of renal transplant patients and its expression correlated to renal histology (Banff and C4d staining) and function. The relevance of this gene was additionally studied in several rat models of allotransplantation and its function analyzed in the relevant cell subtype from healthy volunteers. TRIB1 was significantly up-regulated in the PBMC of CR patients compared to those with stable renal function (Figure 1). It was also significantly higher in CR patients with C4d+ve vs. C4d-ve biopsies and showed a tendency to increase with worsening Banff scores (Figure 1). TRIB1 was also increased in the graft biopsies of patients with chronic humoral rejection vs. other causes of late graft failure such as tubular atrophy/interstitial fibrosis (TA/IF) of unknown etiology, calcineurin inhibitor (CNI) toxicity, or transplant glomerulopathy (figure 1).

Interestingly, TRIB1 was also significantly upregulated in heart allografts presenting CR vs. tolerance in the rat (Figure 2A). TRIB1 was also shown to be a marker of activated monocytes and mature dendritic cells (Figures 2B and C). The present study is thus the first one to show that TRIB1 is significantly upregulated in the peripheral blood of patients with CR. The inventors herein show that TRIB1 is a peripheral blood marker of CR.

Given the high expression of TRIB1 by monocytes and B cells, the blood formulae of the patients were analyzed in more detail. No significant correlation was found between total monocyte numbers and TRIB1 (p = 0.23), indicating that the differences in TRIB1 observed in total PBMC do not simply reflect monocyte numbers.

Finally, because CHR is likely to be associated with chronic activation of endothelial cells (EC), the inventors studied TRIB1 mRNA accumulation in primary human renal artery-derived EC.The inventors showed that TRIB1 is also a marker of activated EC, being upregulated upon activation with the pro-inflammatory cytokine TNFα over 6, 12 and 24hrs.

Thus, TRIB1 is a marker of antigen presenting cells where it is regulated differentially according to both cell and activation type.

Monocytes/macrophages and dendritic cells play a pathogenic role in a number of autoimmune inflammatory diseases including those of the joints and of the brain. Since they found TRIB1 to be expressed primarily by monocytes/dendritic cells, the inventors additionally analyzed TRIB1 expression in the PBMC of patients suffering from two such diseases: Spondyloarthropathies (SpA) and Multiple Sclerosis (MS).

TRIB1 was shown to be a marker of chronic inflammation since it is significantly upregulated in the blood of patients suffering from Spondyarthropathy and in a subgroup of patients suffering from relapsing Multiple Sclerosis (See Examples 2, 3 below).

### Example 2: Spondyloarthropathies

SpA is a group of chronic inflammatory arthritides classified according to common features of peripheral and spinal arthritis.

To measure TRIB1 in the peripheral blood of patients with SpA compared to healthy individuals, PBMC were prepared from the peripheral blood of 10 patients with clinically defined SpA, and 10 healthy age-matched controls. The PBMC were subsequently subjected to RNA extraction and TRIB1 expression was measured by quantitative RT-PCR using commercialized TaqMan primers and probes. TRIB1 was shown to be significantly upregulated in the blood of patients suffering from Spondylarthropathy (Figure 3A).

### Example 3: Multiple sclerosis

Multiple sclerosis (MS) is an inflammatory demyelinating disease characterized by immune abnormalities in the central nervous system (CNS) as well as in the periphery.

To measure TRIB1 in the peripheral blood of patients with MS compared to healthy individuals, PBMC were prepared from the peripheral blood of 6 patients with clinically defined MS during relapse, and 5 healthy age-matched controls. RNA extraction and TRIB1 expression was measured using commercialized TaqMan primers and probes.

TRIB1 was shown to be significantly upregulated in the blood of a sub-group of patients suffering from relapsing Multiple Sclerosis (Figure 3B).

The following items are described as part of the invention.
Item 1. An *in vitro* method of diagnosis and/or prognosis of an inflammatory condition, which method comprises determining the expression level of TRIB1 in a biological sample of a patient, wherein a higher expression of TRIB1 in the patient, compared to a reference value, is indicative of an inflammatory condition.
Item 2. The method according to item 1, wherein the biological sample is blood, plasma or a tissue biopsy.
Item 3. The method of item 2, wherein the biological sample is peripheral blood.
Item 4. The method according to item 1, wherein the inflammatory condition is graft rejection.
Item 5. The method of item 4, wherein the graft rejection is an allograft rejection.
Item 6. The method according to item 4, wherein the graft rejection is a kidney rejection.
Item 7. The method according to item 6, wherein the graft rejection is chronic rejection in clinical renal transplantation.
Item 8. The method according to item 1, wherein the inflammatory condition is joint inflammation.
Item 9. The method according to item 1, wherein the inflammatory condition is multiple sclerosis.
Item 10. The method according to item 1, wherein the inflammatory condition is inflammatory bowel disease.
Item 11. The method according to item 1, wherein the expression level is determined by measuring the level of TRIB1 mRNA in the biological sample.
Item 12. The method of item 11, which comprises the steps of providing total RNAs extracted from PBMCs obtained from a blood sample or a tissue biopsy of the patient, and subjecting the RNAs to amplification and hybridization to specific probes.
Item 13. The method of item 11, wherein the expression level is determined by real-time quantitative or semi-quantitative RT-PCR.
Item 14. The method according to item 1, wherein the expression level is determined by measuring the level of TRIB1 protein in the biological sample.
Item 15. The method according to item 14, wherein the expression level is determined by an immunoassay of TRIB1 protein.
Item 16. A method for screening compounds useful for treating an inflammatory condition, which method comprises determining the ability of a test compound to modulate the expression or activity of TRIB1.
Item 17. The method of item 16, wherein the test compound that inhibits the expression or activity of TRIB1 is a candidate for treating an inflammatory condition.
Item 18. The method of item 16, wherein the inflammatory condition is graft rejection.
Item 19. The method of item 16, wherein the graft rejection is an allograft rejection.
Item 20. The method according to item 18, wherein the graft rejection is a kidney rejection.
Item 21. The method according to item 20, wherein the graft rejection is chronic rejection in clinical renal transplantation.
Item 22. The method according to item 16, wherein the inflammatory condition is joint inflammation.
Item 23. The method according to item 16, wherein the inflammatory condition is multiple sclerosis.
Item 24. The method according to item 16, wherein the inflammatory condition is inflammatory bowel disease.
Item 25. A method of treating an inflammatory condition in a patient in need thereof, which method comprises modulating TRIB1 expression or activity in said patient.
Item 26. The method of item 25, which comprises administering to a patient in need thereof a modulator of TRIB1 expression or activity selected from the group consisting of an antagonist of TRIB1, an antisense or a RNAi of TRIB1, and an antibody or a fragment or a derivative thereof specific to a TRIB1 polypeptide.
Item 27. The method according to item 26, wherein the inflammatory condition is graft rejection.
Item 28. The method of item 27, wherein the graft rejection is an allograft rejection.
Item 29. The method according to item 27, wherein the graft rejection is a kidney rejection.
Item 30. The method according to item 29, wherein the graft rejection is chronic rejection in clinical renal transplantation.
Item 31. The method according to item 25, wherein the inflammatory condition is joint inflammation.
Item 32. The method according to item 25, wherein the inflammatory condition is multiple sclerosis.
Item 33. The method according to item 25, wherein the inflammatory condition is inflammatory bowel disease.

### Bibliography

- Akalin E, Hendrix RC, Polavarapu RG, Pearson TC, Neylan JF, Larsen CP, et al. Gene expression analysis in human renal allograft biopsy samples using high-density oligoarray technology. Transplantation 2001;72(5):948-53.
- Baeten D, Louis S, Braud C, Braudeau C, Ballet C, Moizant F, et al. Phenotypically and functionally distinct CD8+ lymphocyte populations in long-term drug-free tolerance and chronic rejection in human kidney graft recipients. J Am Soc Nephrol 2006;17(1):294-304.
- Brouard S, Dupont A, Giral M, Louis S, Lair D, Braudeau C, et al. Operationally tolerant and minimally immunosuppressed kidney recipients display strongly altered blood T-cell clonal regulation. Am J Transplant 2005;5(2):330-40.
- Donauer J, Rumberger B, Klein M, Faller D, Wilpert J, Sparna T, et al. Expression profiling on chronically rejected transplant kidneys. Transplantation 2003;76(3):539-47
- Flechner SM, Kurian SM, Solez K, Cook DJ, Burke JT, Rollin H, et al. De novo kidney transplantation without use of calcineurin inhibitors preserves renal structure and function at two years. Am J Transplant 2004a;4(11):1776-85.
- Flechner SM, Kurian SM, Head SR, Sharp SM, Whisenant TC, Zhang J, et al. Kidney transplant rejection and tissue injury by gene profiling of biopsies and peripheral blood lymphocytes. Am J Transplant 2004b;4(9):1475-89.
- Kiss-Toth E, Bagstaff SM, Sung HY, Jozsa V, Dempsey C, Caunt JC, et al. Human tribbles, a protein family controlling mitogen-activated protein kinase cascades. J Biol Chem 2004;279(41):42703-8.
- Louis S, Braudeau C, Giral M, Dupont A, Moizant F, Robillard N, et al. Contrasting CD25hiCD4+T cells/FOXP3 patterns in chronic rejection and operational drug-free tolerance. Transplantation 2006;81 (3):398-407.
- Mauiyyedi S, Pelle PD, Saidman S, Collins AB, Pascual M, Tolkoff-Rubin NE, Williams WW, Cosimi AA, Schneeberger EE and Colvin RB: Chronic humoral rejection: identification of antibody-mediated chronic renal allograft rejection by C4d deposits in peritubular capillaries. J Am Soc Nephrol 12: 574-582, 2001
- Scherer A, Krause A, Walker JR, Korn A, Niese D, Raulf F. Early prognosis of the development of renal chronic allograft rejection by gene expression profiling of human protocol biopsies. Transplantation 2003;75(8):1323-30.

## Claims

1. An *in vitro* method of diagnosis and/or prognosis of an inflammatory condition, which method comprises determining the expression level of TRIB1 in a biological sample of blood or plasma of a patient, wherein a higher expression of TRIB1 in the patient, compared to a reference value, is indicative of an inflammatory condition.

2. The method of claim 1, wherein the biological sample is peripheral blood.

3. The method according to claim 1, wherein the inflammatory condition is joint inflammation.

4. The method according to claim 1, wherein the inflammatory condition is multiple sclerosis.

5. The method according to claim 1, wherein the inflammatory condition is inflammatory bowel disease.

6. The method according to claim 1, wherein the inflammatory condition is spondylarthropathy.

7. The method according to claim 1, wherein the expression level is determined by measuring the level of TRIB1 mRNA in the biological sample.

8. The method of claim 7, which comprises the steps of providing total RNAs extracted from PBMCs obtained from a blood sample of the patient, and subjecting the RNAs to amplification and hybridization to specific probes.

9. The method of claim 7, wherein the expression level is determined by real-time quantitative or semi-quantitative RT-PCR.

10. The method according to claim 1, wherein the expression level is determined by measuring the level of TRIB1 protein in the biological sample, preferably by an immunoassay of TRIB1 protein.


SEQUENCE LISTING

<110>  INSERM

<120>  Methods for diagnosing and treating graft rejection and
       inflammatory conditions

<130>  B0472WO

<160>  2

<170>  PatentIn version 3.3

<210>  1
<211>  3658
<212>  DNA
<213>  Homo sapiens


<220>
<221>  CDS
<222>  (583)..(1701)

<400>  1
aaggaggagt gcactggccg ggatcggtgc agcgctcaca ctcactcaca gtcactctct      60

ctgagcgcgt ctcgctcgct ctcatacacg cccggagccc aggagcgctc aggatcccga     120

gcgccgcgaa aaagttcccc cggcttttgc tggagactca tcgttttggg aagtgcattt     180

gcttcgtggc tccgccgagc ctgctgaatc ctgtcctcgc ggcacgggac cccgggatcg     240

ctgaccgctg ccgccgccgc ctctgcctcc cggactatcg gcagcctcgg caacaatagt     300

ggcggccgcc cccagcgagg ctccgggagc ccttgcctgc gggggtccgg ggactcgagc     360

cggcctccgc ctcccggacg cacagccagc gtggtccccg cgtgcaacgc gagcgccggg     420

gagtggctcc tgctttgccc ctcgtggggg ccgagccaag accagtctgc aaactccatc     480

ccgccggctg gaagaagtcg cggagccggc accaaacccg cagcgtcttc ccgcgcggat     540

cccgggactt aaaaagccgg ggccaccccg gcccaggacg gg atg cgg gtc ggt        594
                                               Met Arg Val Gly
                                               1

ccg gtg cgc tct gcc atg agc ggc gcc tcg cag ccc cgc ggc ccg gcc       642
Pro Val Arg Ser Ala Met Ser Gly Ala Ser Gln Pro Arg Gly Pro Ala
5                   10                  15                  20

ctg ctc ttc cca gcc acc cga ggc gtc ccg gcc aaa cgc ctg ctg gac       690
Leu Leu Phe Pro Ala Thr Arg Gly Val Pro Ala Lys Arg Leu Leu Asp
                25                  30                  35

gcc gac gac gcg gcg gct gtg gcg gcc aag tgc ccg cgc ctc tcc gag       738
Ala Asp Asp Ala Ala Ala Val Ala Ala Lys Cys Pro Arg Leu Ser Glu
            40                  45                  50

tgc tcc agc ccc ccg gac tac ctc agc ccc ccc ggc tcg ccc tgc agc       786
Cys Ser Ser Pro Pro Asp Tyr Leu Ser Pro Pro Gly Ser Pro Cys Ser
        55                  60                  65

ccg cag ccc ccg cct gcc gct ccg ggg gcc ggc gga ggc tcc ggg agc     834
Pro Gln Pro Pro Pro Ala Ala Pro Gly Ala Gly Gly Gly Ser Gly Ser
    70                  75                  80

gcg ccg ggg ccc agc cgc atc gcc gac tac ctg ctg ctg ccc cta gcc     882
Ala Pro Gly Pro Ser Arg Ile Ala Asp Tyr Leu Leu Leu Pro Leu Ala
85                  90                  95                  100

gag cgc gag cat gtg tcc cgg gcg ctg tgc atc cac act gga cgc gag     930
Glu Arg Glu His Val Ser Arg Ala Leu Cys Ile His Thr Gly Arg Glu
                105                 110                 115

ctg cgc tgc aag gtg ttt ccc att aaa cac tac cag gac aaa atc agg     978
Leu Arg Cys Lys Val Phe Pro Ile Lys His Tyr Gln Asp Lys Ile Arg
            120                 125                 130

cct tac atc cag ctg cca tcg cac agc aac att act ggc att gtg gaa     1026
Pro Tyr Ile Gln Leu Pro Ser His Ser Asn Ile Thr Gly Ile Val Glu
        135                 140                 145

gtg atc ctt ggg gaa acc aag gcc tat gtc ttc ttt gag aag gac ttt     1074
Val Ile Leu Gly Glu Thr Lys Ala Tyr Val Phe Phe Glu Lys Asp Phe
    150                 155                 160

ggg gac atg cac tcc tat gtg cga agc cgg aag agg ctg cgg gaa gag     1122
Gly Asp Met His Ser Tyr Val Arg Ser Arg Lys Arg Leu Arg Glu Glu
165                 170                 175                 180

gaa gcc gcc cgg ctc ttc aag cag att gtc tcc gcc gtc gcc cac tgc     1170
Glu Ala Ala Arg Leu Phe Lys Gln Ile Val Ser Ala Val Ala His Cys
                185                 190                 195

cac cag tca gcc atc gtg ctg ggg gac ctg aag ctt agg aag ttc gtc     1218
His Gln Ser Ala Ile Val Leu Gly Asp Leu Lys Leu Arg Lys Phe Val
            200                 205                 210

ttc tcc acg gag gag aga acc cag ctt aga cta gaa agt cta gaa gac     1266
Phe Ser Thr Glu Glu Arg Thr Gln Leu Arg Leu Glu Ser Leu Glu Asp
        215                 220                 225

aca cac ata atg aag ggg gaa gat gat gct ttg tca gac aaa cat ggc     1314
Thr His Ile Met Lys Gly Glu Asp Asp Ala Leu Ser Asp Lys His Gly
    230                 235                 240

tgc cca gcc tac gtg agc cct gag atc ctc aac acc act ggg acc tac     1362
Cys Pro Ala Tyr Val Ser Pro Glu Ile Leu Asn Thr Thr Gly Thr Tyr
245                 250                 255                 260

tcc gga aag gct gcg gac gtt tgg agc ctg ggg gtg atg ctc tac acc     1410
Ser Gly Lys Ala Ala Asp Val Trp Ser Leu Gly Val Met Leu Tyr Thr
                265                 270                 275

ctt ctg gtt gga cga tac ccc ttc cat gac tca gac ccc agt gcc ctt     1458
Leu Leu Val Gly Arg Tyr Pro Phe His Asp Ser Asp Pro Ser Ala Leu
            280                 285                 290

ttc tcc aaa att cgg cgt gga cag ttc tgc att cct gag cac att tcc     1506
Phe Ser Lys Ile Arg Arg Gly Gln Phe Cys Ile Pro Glu His Ile Ser
        295                 300                 305

ccc aaa gcc agg tgc ctc att cgc agc ctc ttg aga cgg gag ccc tcc     1554
Pro Lys Ala Arg Cys Leu Ile Arg Ser Leu Leu Arg Arg Glu Pro Ser
    310                 315                 320

gag aga ctc act gcc ccc gag atc cta ctg cac ccc tgg ttt gag tcc     1602
Glu Arg Leu Thr Ala Pro Glu Ile Leu Leu His Pro Trp Phe Glu Ser
325                 330                 335                 340

gtc ttg gaa ccc ggg tac atc gac tca gaa ata gga act tca gac cag     1650
Val Leu Glu Pro Gly Tyr Ile Asp Ser Glu Ile Gly Thr Ser Asp Gln
                345                 350                 355

att gtt cca gag tac cag gag gac agt gac att agt tcc ttc ttc tgc     1698
Ile Val Pro Glu Tyr Gln Glu Asp Ser Asp Ile Ser Ser Phe Phe Cys
            360                 365                 370

taa tccccaaaac ctcagaaacc tcataattct taacacctgg catttccatt          1751

tctaaagatg gacaggccct ttggcgtggt accaaccaga taatgactgc atcaggatga   1811

aagctgctga actcggcatg gcgcctcctc ttctctgttg ggatgagtga ctttattgat   1871

ttgagcagca tatgctgtga ttggctgccc tgcaaatttg tttcccttaa ggaaccctca   1931

ccaactatct ctgctggatt tgggagttcc gcatcttttg tggagggcag agtatggaca   1991

tcttacaccc ggtggtcaag tgtgtaataa acttgagcat tcgaatggga gaaaaagcaa   2051

atcgcacaat gacatatttt gagtaataac cgtatttttc acagggtgac aaattgggcc   2111

aataaatctg ccatctttga actcatcttt ggtggctaga ctgctacggc agcttctctg   2171

atgggaaagt tccttttttg gcttaacact caccctttct tcacactcac atttaccaat   2231

gactctgctc cgtttttgga gcagactgtt ttaagttgct caggagcctg atggaaccat   2291

gaaccgagac tcttctctgt ttcctgccaa gacctcatct gcactaatgc cttctccctg   2351

accttgacac ttcccccttt agctataaaa gcacttacca gccgaacgtg gaacagtatc   2411

acaaaagatt ccatctccca acgatttcag aactctgagc tcagagagac tccagatttt   2471

aaaaaataat ttgagtgctt ggaaactatt agctttttaa gttccttcca aatatgttag   2531

tacctaccct ttactttttc cccaagacca tctcagggtg gagcattctg tctaagagaa   2591

gaaagataag gaggctccca cccacctctc ccaagagcag acattaaaca tctttgtgct   2651

ttgaagagag tgaattttgg atagtcttgt gattctcaga ctaacttcca gaattatact   2711

ttaacccctc ccagatatgg tccgcctttg gcattgtgtg tacatctgca gttttgcatg   2771

gtgggttgtt aatatttcaa atgtgtggtt tatgaatacg tctgtataat cggcttctgg   2831

agtgaaacag caaaccccaa atcttcaaag ttggaaggaa ctttaaaaat catccggtcc   2891

aatctctttc ctctttctgc cacctcccaa ggcagaaatc ccctcttcag cttcttttgt   2951

aggtgggaat ccagcctctg ttagatatgt ccagagatgg aaactcactc ccctacaaaa   3011

gatggagctt aatggagaaa ttgcaacttt cattaaaaaa caaattcaga tgaaatatca   3071

gtaactgtct tggacagtgc tgaaatcagg tggttaaacg ggtaaacaaa atatactgta 3131

ttttgagaaa tggcacaaaa acaggcagtc atctttaagg gctatgccta ggcaaactac 3191

taacatgcat tgtgagaatg ccgtgtatac ctcacgtact gtgtactttg tacatatatt 3251

ttacctttta tacctatgtt cgattttgtt ttgttttgtt ttgttctggc tttgaggctt 3311

gttttgttgt ctgtgtctgt ctgaataacc tgcgtgtcta aaaccacgtg aaatgtgaat 3371

gattattggc aatattacct tgacagaatc atgggacttt gagaagaggg aggacagagg 3431

cctctgtcgc actaacgctc tcgtggttgc tcgactgttg tatctgtgat acattatccg 3491

actaaggact ctgggctggc agggccttct gccgggaaag ctagaaacac taggttcttc 3551

ctgtacatac gtgtatatat gtgaacagtg agatggccgt ttctgacttg tagagaaatt 3611

ttaataaacc tggtttcgta aaaaaaaaaa aaaaaaaaaa aaaaaaa 3658

<210> 2
<211> 372
<212> PRT
<213> Homo sapiens

<400> 2

Met Arg Val Gly Pro Val Arg Ser Ala Met Ser Gly Ala Ser Gln Pro
1               5                   10                  15

Arg Gly Pro Ala Leu Leu Phe Pro Ala Thr Arg Gly Val Pro Ala Lys
            20                  25                  30

Arg Leu Leu Asp Ala Asp Asp Ala Ala Ala Val Ala Ala Lys Cys Pro
        35                  40                  45

Arg Leu Ser Glu Cys Ser Ser Pro Pro Asp Tyr Leu Ser Pro Pro Gly
    50                  55                  60

Ser Pro Cys Ser Pro Gln Pro Pro Pro Ala Ala Pro Gly Ala Gly Gly
65                  70                  75                  80

Gly Ser Gly Ser Ala Pro Gly Pro Ser Arg Ile Ala Asp Tyr Leu Leu
                85                  90                  95

Leu Pro Leu Ala Glu Arg Glu His Val Ser Arg Ala Leu Cys Ile His
            100                 105                 110

Thr Gly Arg Glu Leu Arg Cys Lys Val Phe Pro Ile Lys His Tyr Gln
        115                 120                 125

Asp Lys Ile Arg Pro Tyr Ile Gln Leu Pro Ser His Ser Asn Ile Thr
    130                 135                 140

Gly Ile Val Glu Val Ile Leu Gly Glu Thr Lys Ala Tyr Val Phe Phe
145                 150                 155                 160

Glu Lys Asp Phe Gly Asp Met His Ser Tyr Val Arg Ser Arg Lys Arg
                165                 170                 175

Leu Arg Glu Glu Glu Ala Ala Arg Leu Phe Lys Gln Ile Val Ser Ala
            180                 185                 190

Val Ala His Cys His Gln Ser Ala Ile Val Leu Gly Asp Leu Lys Leu
        195                 200                 205

Arg Lys Phe Val Phe Ser Thr Glu Glu Arg Thr Gln Leu Arg Leu Glu
    210                 215                 220

Ser Leu Glu Asp Thr His Ile Met Lys Gly Glu Asp Asp Ala Leu Ser
225                 230                 235                 240

Asp Lys His Gly Cys Pro Ala Tyr Val Ser Pro Glu Ile Leu Asn Thr
                245                 250                 255

Thr Gly Thr Tyr Ser Gly Lys Ala Ala Asp Val Trp Ser Leu Gly Val
            260                 265                 270

Met Leu Tyr Thr Leu Leu Val Gly Arg Tyr Pro Phe His Asp Ser Asp
        275                 280                 285

Pro Ser Ala Leu Phe Ser Lys Ile Arg Arg Gly Gln Phe Cys Ile Pro
    290                 295                 300

Glu His Ile Ser Pro Lys Ala Arg Cys Leu Ile Arg Ser Leu Leu Arg
305                 310                 315                 320

Arg Glu Pro Ser Glu Arg Leu Thr Ala Pro Glu Ile Leu Leu His Pro
                325                 330                 335

Trp Phe Glu Ser Val Leu Glu Pro Gly Tyr Ile Asp Ser Glu Ile Gly
            340                 345                 350

Thr Ser Asp Gln Ile Val Pro Glu Tyr Gln Glu Asp Ser Asp Ile Ser
        355                 360                 365

Ser Phe Phe Cys

    370